(19) Europäisches Patentamt
European Patent Office
Office européen des brevets

(11) Veröffentlichungsnummer: **0 036 652**
**A2**

(12) # EUROPÄISCHE PATENTANMELDUNG

(21) Anmeldenummer: **81102131.0**

(22) Anmeldetag: **20.03.81**

(51) Int. Cl.³: **C 07 D 501/36,** A 61 K 31/545 // C07D253/06, C07D249/12, C07C131/00

(30) Priorität: **25.03.80 CH 2326/80**
**15.01.81 CH 245/81**

(43) Veröffentlichungstag der Anmeldung: **30.09.81**
**Patentblatt 81/39**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Montavon, Marc, Dr., Realpstrasse 72, CH-4054 Basel (CH)**
Erfinder: **Reiner, Roland, Dr., Rheinfelderstrasse 8, CH-4058 Basel (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) **Cephalosporinderivate, entsprechende Präparate, die Verwendung dieser Produkte bei der Behandlung von Krankheiten, die Herstellung der Wirkstoffe sowie dabei anfallende Zwischenprodukte.**

(57) Cephalosporinderivate der allgemeinen Formel

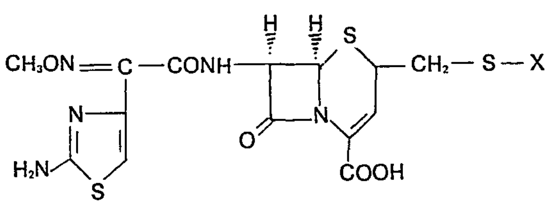

I

in der X eine der Gruppen

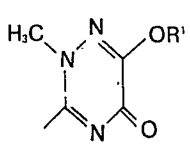

(a)

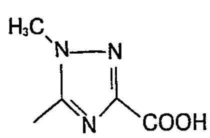

(b)

worin R' niederes Alkyl oder (zusammen mit dem Sauerstoff) eine leicht hydrolysierbare Äthergruppe darstellt, ist sowie leicht hydrolysierbare Ester der Verbindungen der Formel I, worin X die Gruppe (b) oder (a) mit R'= niederes Alkyl bedeutet und Salze dieser Verbindungen und Hydrate der Verbindungen der Formel I bzw. von deren Estern und Salzen.

Ferner auch entsprechende Präparate, die Verwendung dieser Produkte bei der Behandlung von Krankheiten, die Herstellung der Wirkstoffe sowie dabei anfallende Zwischenprodukte.

EP 0 036 652 A2

0036652

F.Hoffmann-La Roche & Co.Aktiengesellschaft,Basel,Schweiz

RAN 4410/143

# Cephalosporinderivate, entsprechende Präparate, die Verwendung dieser Produkte bei der Behandlung von Krankheiten, die Herstellung der Wirkstoffe sowie dabei anfallende Zwischenprodukte.

Die vorliegende Erfindung betrifft neue Cephalosporinderivate der allgemeinen Formel

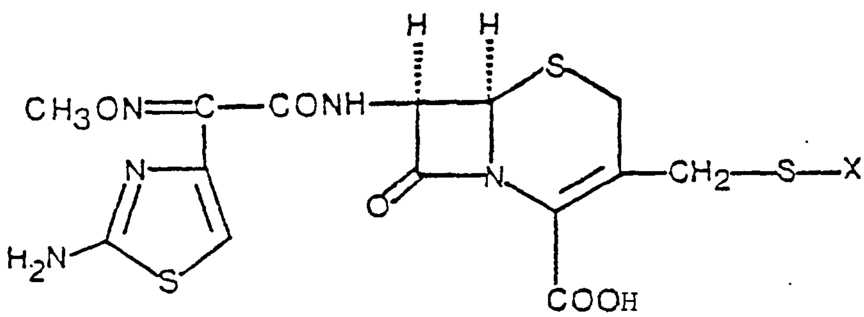

I

in der X eine der Gruppen

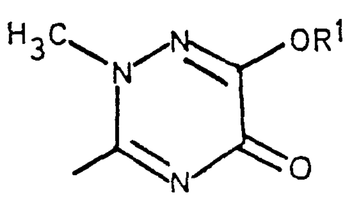

(a)

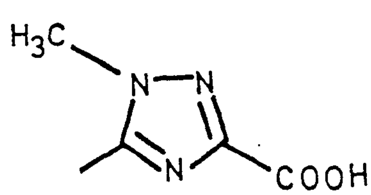

(b)

Mn/1 5.1.81

0036652

worin $R^1$ niederes Alkyl oder (zusammen mit dem Sauerstoff) eine leicht hydrolysierbare Aethergruppe darstellt, ist sowie leicht hydrolysierbare Ester der Verbindungen der Formel I, worin X die Gruppe (b) oder (a) mit $R^1$=niederes Alkyl bedeutet und Salze dieser Verbindungen und Hydrate der Verbindungen der Formel I bzw. von deren Estern und Salzen.

$R^1$ in der Gruppe (a) kann niederes Alkyl darstellen, vorzugsweise eine geradkettige oder verzweigte Alkylgruppe mit 1-7 Kohlenstoffatomen, wie Aethyl, n-Propyl, Isopropyl, n-Butyl, Isobutyl, sec-Butyl, t-Butyl, n-Pentyl oder, insbesondere, Methyl. $R^1$ kann (zusammen mit dem Sauerstoff) ebenfalls eine leicht hydrolysierbare Aethergruppe darstellen. So kann $R^1$ beispielsweise bedeuten: Niederes Alkanoyloxyalkyl, z.B. Acetoxymethyl, Pivaloyloxymethyl, 1-Acetoxyäthyl und 1-Pivaloyloxyäthyl; niederes Alkoxycarbonyloxyalkyl, z.B. Methoxycarbonyloxymethyl, 1-Aethoxycarbonyloxyäthyl und 1-Isopropoxycarbonyloxyäthyl; Lactonylgruppen, z.B. Phthalidyl und Thiophthalidyl; niederes Alkoxymethyl, z.B. Methoxymethyl; und niederes Alkanoylaminomethyl, z.B. Acetamidomethyl.

Als leicht hydrolysierbare Ester der Verbindungen der Formel I sind Verbindungen der Formel I zu verstehen, worin mindestens eine Carboxygruppe in Form einer leicht hydrolysierbaren Estergruppe vorliegt. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl- und 1-Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein. Die Ester können Monoester oder auch Diester sein. Die zweite Esterbildung

kann in Verbindung mit dem Carboxyrest der 3-Carboxy-1-methyl-1H-1,2,4-triazol-5-ylgruppe X (Gruppe (b)) auftreten.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Monosalze oder auch Disalze sein. Die zweite Salzbildung kann in Verbindungen mit dem Carboxyrest der 3-Carboxy-1-methyl-1H-1,2,4-triazol-5-ylgruppe X (Gruppe (b)) auftreten.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Monoaryl-sulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die Verbindungen der Formel I (einschliesslich deren Salze und leicht hydrolysierbare Ester) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der syn-isomeren Form

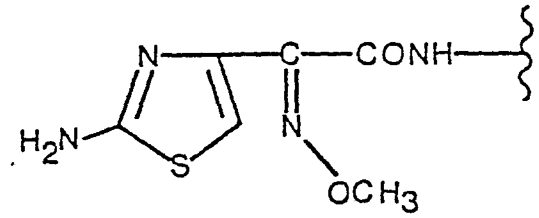

oder in der anti-isomeren Form

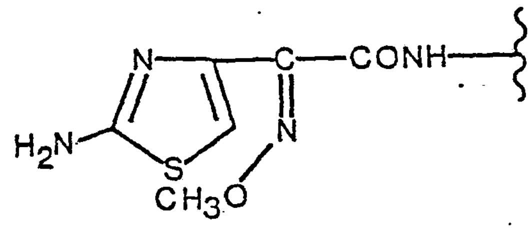

bzw. als Gemische dieser beiden Formen vorliegen. Bevorzugt ist die syn-isomere Form bzw. Gemische, in denen die syn-isomere Form überwiegt.

Bevorzugte Produkte sind

- (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure
  (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]oct-2-en-2-carbonsäure

- (6R,7R)-7-[2-(2-Amino-4-thiazolyl)-2-((Z)-methoxyimino)acetamido]-3-[[[2,5-dihydro-2-methyl-5-oxo-6-[(pivaloyloxy)methoxy]-as-triazin-3-yl]thio]methyl]-5-oxo-4-thia-1-azabicyclo[4.2.0]oct-2-en-2-carbonsäure

und deren Salze sowie die entsprechenden Hydrate.

Die obigen Cephalosporinderivate können erfindungsgemäss dadurch hergestellt werden, dass man

a) in einer Verbindung der allgemeinen Formel

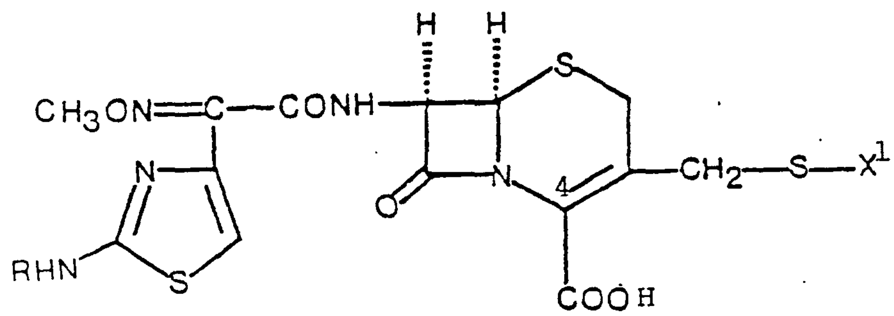 II

in der $X^1$ die gleiche Bedeutung wie X in Formel I hat, wobei die Carboxygruppe des Restes (b) geschützt sein kann, R eine abspaltbare Schutzgruppe darstellt und die Carboxygruppe in 4-Stellung des Cephalosporingerüsts in geschützter Form vorliegen kann,

die Schutzgruppe R, ggfs. auch allenfalls vorhandene Carboxyschutzgruppen, abspaltet, oder dass man

b) ein Halogenid der allgemeinen Formel

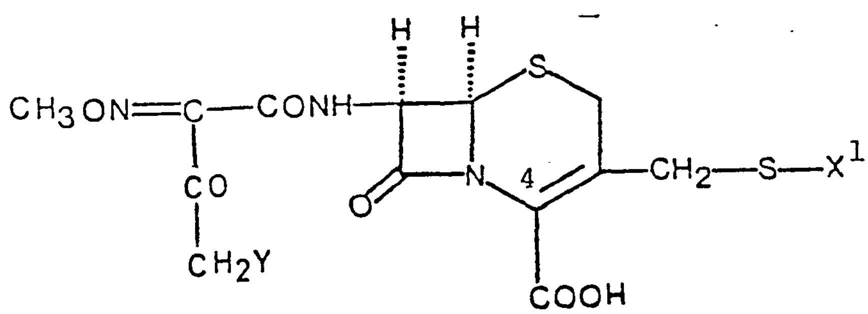 III

in der $X^1$ die oben gegebene Bedeutung hat und Y ein Halogenatom darstellt und die Carboxygruppe in 4-Stellung des Cephalosporingerüsts in geschützter Form vorliegen kann,

mit Thioharnstoff umsetzt, oder dass man

c) zur Herstellung eines leicht hydrolysierbaren Esters einer Verbindung der Formel I eine Carbonsäure der Formel I einer entsprechenden Veresterung unterwirft, oder dass

man

d) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

In den Ausgangsverbindungen der Formeln II und III vorhandene Carboxygruppen können wahlweise geschützt sein, z.B. durch Veresterung zu einem leicht spaltbaren Ester, wie einem Silylester, z.B. dem Trimethylsilylester. Es kommen auch die oben erläuterten, leicht hydrolysierbaren Ester in Betracht. Die Carboxygruppen können auch durch Salzbildung mit einer anorganischen oder tertiären organischen Base, wie Triäthylamin, geschützt werden. Mögliche R-Schutzgruppen sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte R-Schutzgruppen sind Chlor-, Brom- und Jodacetyl, insbesondere Chloracetyl. Letztere Schutzgruppen können durch Behandeln mit Thioharnstoff abgespalten werden.

Die Ausgangsverbindungen der Formel II können z.B. durch N-Acylierung der entsprechenden 7-Aminoverbindung hergestellt werden, und zwar indem man eine Verbindung der allgemeinen Formel

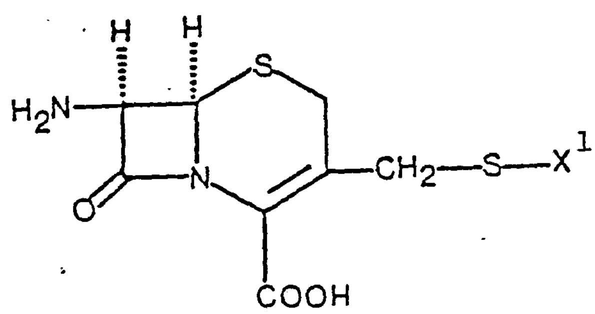

IV

in der $X^1$ die oben gegebene Bedeutung hat und die Carboxygruppe und/oder die Aminogruppe in geschützter Form vorliegen kann,
mit einer Säure der allgemeinen Formel

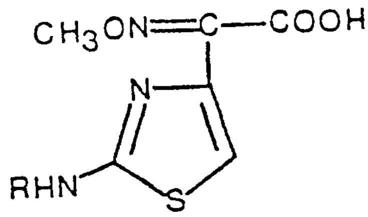

V

in der R die oben gegebene Bedeutung hat, oder mit einem reaktionsfähigen funktionellen Derivat dieser Säure umsetzt und allenfalls vorhandene Carboxyschutzgruppen gegebenenfalls abspaltet.

Die in der 7-Aminoverbindung der Formel IV vorhandenen Carboxygruppen können wahlweise geschützt sein, und zwar in der oben für die herzustellende Ausgangsverbindung der Formel II erläuterten Weise. Die Aminogruppe der Verbindung der Formel IV kann z.B. durch eine Silylschutzgruppe, wie Trimethylsilyl, geschützt sein.

Als reaktionsfähige funktionelle Derivate von Säuren der Formel V kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel IV mit der Säure der Formel V oder einem reaktionsfähigen funktionellen Derivat davon kann in an sich bekannter Weise durchgeführt werden. So kann man z.B. eine freie Säure der Formel V mit einem der erwähnten Ester entsprechend Formel IV mittels eines Carbodiimids, wie Dicylohexylcarbodiimid, in einem inerten Lösungsmittel, wie Aethylacetat, Acetonitril, Dioxan, Chloroform, Methylenchlorid, Benzol oder Dimethylformamid, kondensieren

und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel IV, z.B. ein Trialkylammoniumsalz, wie das Triäthylammoniumsalz, mit einem reaktionsfähigen funktionellen Derivat einer Säure der Formel V wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel V mit dem Amin der Formel IV umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggfs. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel IV wird in wasserfreiem Medium gearbeitet.

Die Umsetzung der 7-Aminoverbindung der Formel IV mit der Säure der Formel V oder einem reaktionsfähigen funktionellen Derivat davon kann zweckmässig bei Temperaturen zwischen etwa -40°C und Zimmertemperatur, beispielsweise bei etwa 0-10°C, erfolgen.

Ausgangsverbindungen der Formel II, worin R nicht monohalogeniert ist, wie in Brom-, Chlor- und Jodacetyl, können auch durch Thiolierung hergestellt werden, und zwar indem man

eine Verbindung der allgemeinen Formel

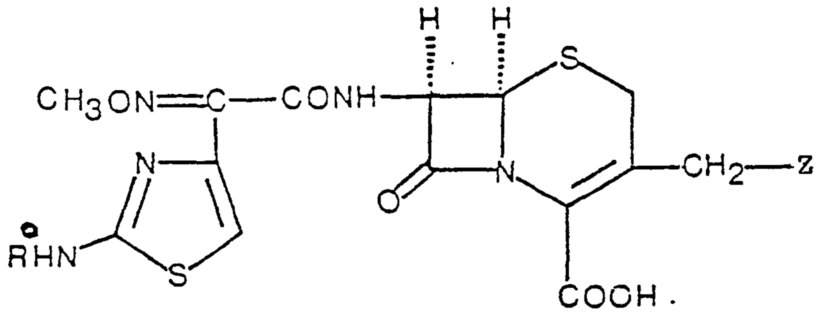

VI

in der R° wie R ist, jedoch nicht monohalogeniert sein kann, Z eine austretende Gruppe darstellt und die Carboxygruppe durch Salzbildung mit einer anorganischen oder tertiären organischen Base geschützt sein kann,

mit einem Thiol der allgemeinen Formel

$$HS-X^1 \quad VII$$

in der $X^1$ die oben gegebene Bedeutung hat,

umsetzt und eine allenfalls vorhandene Carboxyschutzgruppe gegebenenfalls abspaltet.

Als austretende Gruppe Z einer Verbindung der Formel VI kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel VI mit dem Thiol der Formel VII kann in an sich bekannter Weise, z.B. bei einer Temperatur zwischen etwa 40 und 80°C, zweckmässig bei etwa 60°C, vorzugsweise in

Wasser oder in einer Pufferlösung mit einem pH von etwa 6 bis 7, vorzugsweise 6,5, durchgeführt werden. Die Carboxygruppe der erhaltenen Verbindungen II kann erwünschtenfalls anschliessend geschützt werden, z.B. durch Salzbildung oder Veresterung.

Die Thiole der Formel VII stehen in tautomerem Gleichgewicht mit den entsprechenden Thionen im Sinne der nachstehenden Formeln

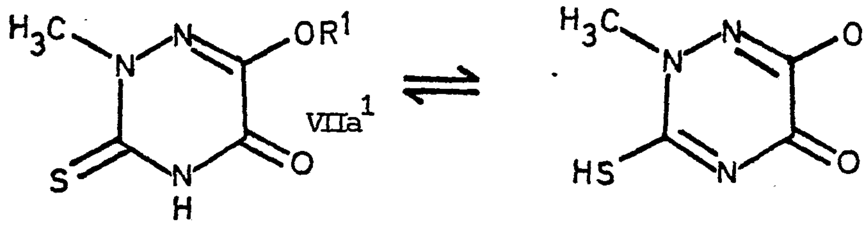

VIIa[1] VIIa[2]

worin $R^1$ die obige Bedeutung hat

bzw.

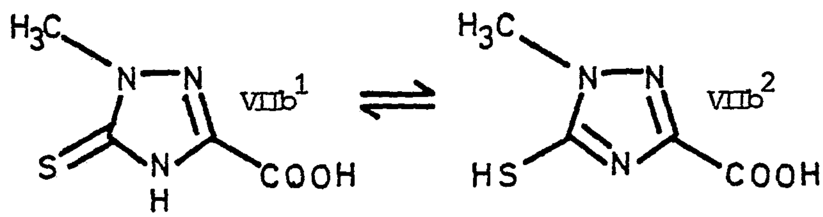

VIIb[1] VIIb[2]

worin die Carboxygruppe geschützt sein kann. Die Herstellung dieser Verbindungen wird in den Beispielen 1, 2 und 3 beschrieben. Zur Herstellung von in 6-Stellung verätherten Thiolen (Thionen) entsprechend der Gruppe (a) wird im allgemeinen die $R^1$-Gruppe eingeführt, indem man ein S-geschütztes Thiol (z.B. durch Benzhydryl) mit dem die $R^1$-Gruppe enthaltenden Halogenid, vorzugsweise dem Jodid, in einem inerten organischen Lösungsmittel in Gegenwart eines säurebindenden Mittels, z.B. Kaliumcarbonat, umsetzt, vorzugsweise bei etwa 10°-50°C, und die Schutzgruppe abspaltet (Benzhydryl kann mit Anisol und Trifluoressigsäure bei Raumtemperatur abgespalten werden).

Gemäss Variante a) des erfindungsgemässen Verfahrens wird die Aminoschutzgruppe R einer Ausgangsverbindung der Formel II abgespalten. Durch saure Hydrolyse abspaltbare Schutzgruppen werden vorzugsweise mit

Hilfe einer niederen Alkancarbonsäure, die ggfs. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa $0^{o}C$ bis $+40^{o}C$. Alkalisch abspaltbare Schutzgruppen werden im allgemeinen mit verdünnter wässriger Lauge bei $0^{o}C$ bis $30^{o}C$ hydrolysiert. Die Chloracetyl-, Bromacetyl- und Jodacetyl-Schutzgruppen können mittels Thioharnstoff in saurem, neutralem oder alkalischem Milieu bei etwa $0-30^{o}C$ abgespalten werden. Hydrogenolytische Abspaltung (z.B. Abspaltung von Benzyl) ist hier ungeeignet, da bei der Hydrogenolyse die Oximfunktion zur Aminogruppe reduziert würde.

Nach Durchführung der Verfahrensvariante a) können, falls erwünscht, allenfalls im Reaktionsprodukt vorhandene Carboxyschutzgruppen abgespalten werden. Wenn die Schutzgruppe eine Silylgruppe darstellt (Silylester), kann diese Gruppe besonders leicht durch Behandeln des Umsetzungsproduktes mit Wasser abgespalten werden. Niedere Alkanoyloxyalkyl-, Alkoxycarbonyloxyalkyl-, Lactonyl-, Alkoxymethyl- und Alkanoylaminomethylester werden vorzugsweise enzymatisch mit Hilfe einer geeigneten Esterase (bei etwa $20-40^{o}C$) abgespalten. Wenn eine Carboxygruppe durch Salzbildung (z.B. mit Triäthylamin) geschützt ist, so kann die Abspaltung dieser salzbildenden Schutzgruppe durch Behandlung mit Säure erfolgen. Als Säure kann hierbei z.B. Salzsäure, Schwefelsäure, Phosphorsäure oder Citronensäure verwendet werden.

Die Carboxyschutzgruppen können in der gleichen Weise wie soeben beschrieben auch vor der Abspaltung der Schutzgruppe R abgespalten werden.

Das erfindungsgemäss eingesetzte Halogenid der Formel III kann z.B. durch Umsetzung einer Verbindung der Formel IV mit einer halogenierten Carbonsäure der allgemeinen Formel

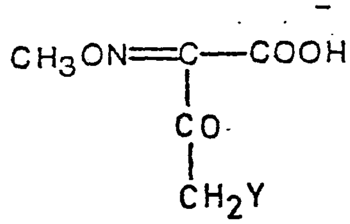

VIII

in der Y ein Halogenatom darstellt,

oder mit einem reaktionsfähigen Derivat dieser Verbindung hergestellt werden. Die halogenierte Carbonsäure der Formel VIII wird entweder in freier Form eingesetzt in Gegenwart eines Kondensationsmittels, z.B. eines N,N'-disubstituierten Carbodiimides, wie N,N'-Dicyclohexylcarbodiimid, oder einer Azolidverbindung, wie N,N'-Carbonyldiimidazol oder N,N'-Thionyldiimidazol oder auch in Form eines Säurehalogenids, wie des Säurechlorids oder -bromids, in Form eines Säureanhydrids, wie eines Säureanhydrids mit einem Kohlensäuremonoester, z.B. mit Monomethyl- oder Monoisopropylcarbonat, oder in Form eines aktivierten Esters, wie des p-Nitrophenylesters, 2,4-Dinitrophenylesters, N-Hydroxysuccinimidesters oder N-Hydroxyphthalimidesters. Die Reaktion erfolgt im allgemeinen in einem inerten organischen Lösungsmittel, z.B. in einem halogenierten Kohlenwasserstoff, wie Chloroform, Dichlormethan, Tetrachlorkohlenstoff, in einem Aether, z.B. Tetrahydrofuran, Dioxan, in Dimethylformamid, Dimethylacetamid, Wasser oder Mischungen davon. Die Reaktionstemperatur liegt vornehmlich im Bereich von etwa -50 bis +40°C, vorzugsweise bei etwa -10 bis +10°C.

Die erfindungsgemässe Umsetzung des Halogenids der Formel III mit Thioharnstoff (Variante b) des erfindungsgemässen Verfahrens) erfolgt vorzugsweise in einem inerten Lösungsmittel, wie z.B. in einem niederen Alkanol, z.B. Aethanol, in einem niederen Keton, wie Aceton, in einem Aether, wie Tetrahydrofuran, Dioxan, in Dimethylformamid, Dimethylacetamid, in Wasser oder in Mischungen davon. Die Reaktionstemperatur liegt im allgemeinen im Bereich von etwa 0°C bis 60°C, vorzugsweise bei Zimmertemperatur. Als Halogenid der Formel III kann das Chlorid, Bromid, Fluorid oder Jodid eingesetzt werden, bevorzugt verwendet man das Chlorid oder das Bromid. Es kann die freie Säure der Formel III eingesetzt werden, wahlweise aber auch ein Salz davon, wobei die gleichen Salze

wie die oben erläuterten Salze der Verbindungen der Formel I in Betracht kommen.

Zur Herstellung der leicht hydrolysierbaren Ester der Carbonsäuren der Formel I gemäss Variante c) wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat, oder einem organischen Amin, wie Triäthylamin, beschleunigt werden. Bei Vorhandensein der Gruppe (b) wird deren Carboxyfunktion ebenfalls verestert, wobei ein leicht hydrolysierbarer Diester erhalten wird. Vorzugsweise verwendet man dabei einen Ueberschuss an dem entsprechenden Halogenid. Die Veresterungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40°C.

Die Herstellung der Salze und Hydrate der Verbindungen der Formel I bzw. der Hydrate dieser Salze kann in an sich bekannter Weise erfolgen, z.B. durch Umsetzung der Carbonsäure der Formel I mit einer äquivalenten Menge der gewünschten Base, zweckmässig in einem Lösungsmittel wie Wasser oder in einem organischen Lösungsmittel, wie Aethanol, Methanol, Aceton und anderen mehr. Bei Verwendung eines zweiten Aequivalents an Base erfolgt Salzbildung auch an der Carboxyfunktion einer allenfalls vorhandenen Gruppe (b), wobei ein Disalz entsteht. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester, Aether oder Salz davon) einer feuchten Atmosphäre, z.B. bei etwa +10°C bis +40°C, ausgesetzt werden.

Die oben verwendeten 7-Aminoverbindungen der Formel IV können ausgehend von einer Verbindung der Formel

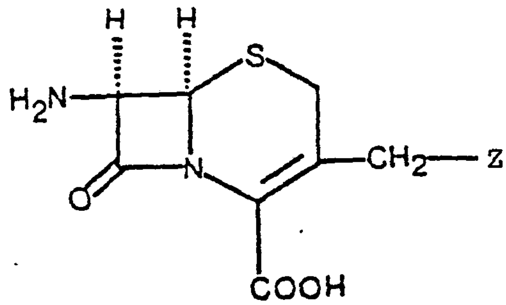

IX

in der Z eine austretende Gruppe darstellt und die Carboxygruppe durch Salzbildung mit einer anorganischen oder tertiären organischen Base geschützt sein kann,

mit einem Thiol der Formel VII hergestellt werden. Die Umsetzung kann unter den gleichen Bedingungen wie denjenigen erfolgen, wie sie für die Umsetzung der Ausgangsverbindungen VI mit VII beschrieben wurden. Andererseits können die Verbindungen der Formel VI ausgehend von einer Verbindung der Formel IX und einer Säure der Formel V oder einem reaktionsfähigen funktionellen Derivat davon unter den gleichen Bedingungen hergestellt werden, wie sie für die Umsetzung der Verbindungen der Formeln IV und V beschrieben wurden.

Die Carboxygruppe und/oder die Aminogruppe der erhaltenen Verbindung IV können erwünschtenfalls anschliessend geschützt werden, z.B. durch Veresterung oder Salzbildung der Carboxygruppe oder durch Silylierung der Aminogruppe.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formeln I und II sowie die entsprechenden leicht hydrolysierbaren Ester und Salze bzw. die Hydrate dieser Produkte sind antibiotisch, insbesondere bakterizid wirksam. Sie besitzen ein breites Wirkungsspektrum gegen Gram-positive Bakterien, z.B. Staphylokokken, und gegen Gram-negative Bakterien, wie z.B. Haemophilus influenzae, Neisseria gonorrhoeae, sowie gegen verschiedene β-Lactamase-bildende Gram-negative Erreger, wie Escherichia coli, Serratia marcescens, Enterobacter cloacae, Pseudomonas aeruginosa, Proteus mirabilis, Proteus vulgaris.

Die Verbindungen der Formel I und II sowie die entsprechenden leicht hydrolysierbaren Ester und Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erwähnten Produkte wurden folgende repräsentative Vertreter getestet:

Produkt A: (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)-thio]methyl/-8-oxo-5-thia-1-azabicyclo-[4.2.0]oct-2-en-2-carbonsäure-Natriumsalz

Produkt B: (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]oct-2-en-2-carbonsäure-Dinatriumsalz

Produkt C: (6R,7R)-7-[2-(2-Amino-4-thiazolyl)-2-((Z)-methoxyimino)acetamido]-3-[[[2,5-dihydro-2-methyl-5-oxo-6-[(pivaloyloxy)methoxy]-as-triazin-3-yl]thio]methyl]-5-oxo-4-thia-1-azabicyclo[4.2.0]oct-2-en-2-carbonsäure-Natriumsalz.

Aktivität in vitro: Mindesthemmkonzentration (µg/ml)

| Krankheitserreger | A | B | C |
|---|---|---|---|
| Escherichia coli *) | 0,04 | 0,08 | 0,10 |
| Serratia marcescens *) | 0,16 | 0,16 | 3,1 |
| Enterobacter cloacae *) | 1,2 | 10 | 25 |
| Proteus mirabilis *) | 0,04 | 0,01 | 0,4 |
| Proteus vulgaris *) | 0,02 | 0,01 | 0,2 |
| Pseudomonas aeruginosa | | | |
| Stamm 1 *) | 40 | 40 | 50 |
| Stamm 2 *) | 80 | 40 | 50 |
| Haemophilus influenzae | 0,01 | 0,01 | 0,04 |
| Neisseria gonorrhoeae | 0,01 | 0,0025 | 0,006 |
| Staphylococcus aureus | 2,5 | 10 | 1,6 |

*) ß-Lactamase bildende Stämme

Verbindungen der Formel I, worin X die Gruppe (b) darstellt, sowie die entsprechenden Ester, Salze und Hydrate zeichnen sich durch eine lange Halbwertzeit aus, d.h. sie bleiben lange im Organismus und können dementsprechend effektiver ihre keimtötende Funktion ausüben. So zeigt z.B. das Dinatriumsalz von (6R,7R)-7-/-2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]-oct-2-en-2-carbonsäure eine Halbwertzeit an der Ratte von 22 Min. nach Verabreichung von 20 mg Substanz pro kg Körpergewicht.

Toxizität

| Prüfsubstanz | A | B | C |
|---|---|---|---|
| $LD_{100}$ mg/kg i.v. | > 500 | | > 500 |
| s.c. | >4000 | | >4000 |
| p.o. | >5000 | >5000 | >5000 |

Die erfindungsgemässen Produkte können als Heilmittel, z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten. Die Verbindungen der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien, wie Wasser oder isotonische Kochsalzlösung, zubereitet. Die leicht hydrolysierbaren Ester der Verbindungen der Formel I und ihre Salze bzw. Hydrate kommen auch für die enterale Verabreichung in Betracht.

## Beispiel 1

Herstellung des Natriumsalzes der (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure

34,5 g Natriumsalz der (6R,7R)-7-/2-[2-(2-Chloracetamido)-4-thiazolyl]-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure werden in 700 ml Wasser zusammen mit 20 g Thioharnstoff gelöst und über Nacht bei 25°C unter Stickstoff-Begasung gerührt, wobei der pH-Wert der Reaktionslösung unter Zugabe von 1N Natronlauge bei 7 gehalten wird. Unter kräftigem Rühren wird der pH-Wert durch Zugabe von 1N Salzsäure auf 4 zurückgestellt. Das dabei ausgefallene Material wird abgenutscht und verworfen. Die gelbe Mutterlauge wird mit 1N Salzsäure auf pH 3 gestellt. Das ausgefallene Produkt wird abgenutscht, mit Wasser, Aethanol und tiefsiedendem Petroläther gewaschen und im Vakuum bei 40°C getrocknet. Man erhält beige-farbene (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo-[4.2.0]oct-2-en-2-carbonsäure, welche, zwecks Ueberführung in das Natriumsalz, in 350 ml Methanol suspendiert und mit 20 ml einer 2N Lösung von 2-Aethylcapronsäure-Natriumsalz in Essigester versetzt wird. Nach ca. 15 Minuten ist eine Lösung entstanden, welche mit 300 ml Aethanol verdünnt wird. Dabei fällt wenig amorphes Material aus, das abgenutscht und verworfen wird. Das Filtrat wird im Vakuum bei 40°C stark eingeengt. Das dabei ausgefallene Material wird abgenutscht, mit Aethanol und tiefsiedendem Petroläther gewaschen, und über Nacht im Hochvakuum bei 40°C getrocknet. Man erhält reines Natriumsalz

0036652

der (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo[4.2.0]-oct-2-en-2-carbonsäure als gelbliches Pulver mit $[\alpha]_D^{25}$ = -140,9° (c = 1 in Wasser).

Die im obigen Verfahren als Ausgangsmaterial verwendete Verbindung kann wie folgt hergestellt werden:

a) Herstellung von Tetrahydro-2-methyl-5,6-dioxo-3-thioxo-as-triazin-1(2H)-carbonsäure-benzylester

39,79 g 1,2,5,6-Tetrahydro-5,6-dioxo-3-mercapto-2-methyl-as-triazin werden in 500 ml Dimethylformamid aufgenommen und mit 35 ml Triäthylamin versetzt. Während 15 Minuten werden 39 ml Benzyloxycarbonylchlorid zugetropft, wobei die Temperatur des Reaktionsgemisches auf 45°C steigt, Triäthylamin-hydrochlorid ausfällt und die Suspension gelb gefärbt wird. Das Gemisch wird 2 1/2 Stunden bei 25°C gerührt und anschliessend im Vakuum bei 70°C eingedampft. Der ölige Eindampfrückstand wird mit 500 ml Wasser während 1 Stunde verrührt, wobei dieser fest wird. Der Festkörper wird abgenutscht und mit 50 ml Wasser gewaschen. Das gelbe Nutschgut wird mit 250 ml Aethanol gut verrührt, abgenutscht, mit Aethanol gewaschen und im Vakuum bei 40°C getrocknet. Man erhält ein farbloses Rohkristallisat, das aus Methanol umkristallisiert wird und farblosen Tetrahydro-2-methyl-5,6-dioxo-3-thioxo-as-triazin-1(2H)-carbonsäure-benzylester vom Schmelzpunkt 150-153°C liefert.

b) Herstellung von 3-[(Diphenylmethyl)thio]-5,6-dihydro-2-methyl-5,6-dioxo-as-triazin-1(2H)-carbonsäure-benzylester

13,2 g Tetrahydro-2-methyl-5,6-dioxo-3-thioxo-as-triazin-1(2H)-carbonsäure-benzylester werden in 500 ml

Aethylacetat gelöst und mit einer Lösung von Diphenyldiazomethan in 90 ml tiefsiedendem Petroläther versetzt. Die anfangs violette Reaktionslösung wird 40 Stunden bei 25°C stehen gelassen, wobei die Farbe blassrot wird. Es werden 3 ml Eisessig zugegeben, und das Gemisch wird nach 1 Stunde im Vakuum bei 40°C eingedampft. Man erhält ein gelbes Oel als Eindampfrückstand. Dieses wird mittels Säulenchromatographie an Kieselgel mit den Elutionsmitteln Benzol, Benzol/Aethylacetat 95:5 und Benzol/Aethylacetat 90:10 aufgetrennt. Die die gewünschte Substanz enthaltenden Fraktionen werden gesammelt und im Vakuum bei 40°C eingedampft. Man erhält nach dem Umkristallisieren aus Aethanol farblosen 3-[(Diphenylmethyl)thio]-5,6-dihydro-2-methyl-5,6-dioxo-as-triazin-1(2H)-carbonsäure-benzylester vom Schmelzpunkt 90-92°C.

c) Herstellung von 3-[(Diphenylmethyl)thio]-1,2-dihydro-2-methyl-as-triazin-5,6-dion

6,9 g 3-[(Diphenylmethyl)thio]-5,6-dihydro-2-methyl-5,6-dioxo-as-triazin-1(2H)-carbonsäure-benzylester werden in 100 ml Aethylacetat gelöst und mit 30 ml einer wässrigen, 7%igen Ammoniak-Lösung bei 25°C während 15 Minuten gut durchgerührt. Die Aethylacetatphase wird abgetrennt und verworfen. Die wässrige Phase wird mit 7 ml konz. Salzsäure versetzt und 30 Minuten im Eisbad gerührt. Die dabei ausgefallene Substanz wird abgenutscht, mit Wasser gewaschen und sofort aus Aethanol umkristallisiert. Man erhält farbloses 3-[(Diphenylmethyl)thio]-1,2-dihydro-2-methyl-as-triazin-5,6-dion vom Schmelzpunkt 180-182°C.

d) Herstellung von 3-[(Diphenylmethyl)thio]-6-methoxy-2-methyl-as-triazin-5(2H)-on

3,9 g 3-[(Diphenylmethyl)thio]-1,2-dihydro-2-methyl-as-triazin-5,6-dion werden in 40 ml Dimethylformamid gelöst und mit 1,68 ml Triäthylamin sowie 4 ml Methyljodid versetzt. Nach 2-stündigem Rühren bei 25°C werden dem Gemisch noch 1,38 g Kaliumcarbonat zugegeben um die Reaktion zu beschleunigen. Nach weiterem 2-stündigem Rühren bei 25°C ist das Ausgangsmaterial vollständig umgesetzt. Die resultierende, rote Suspension wird auf Wasser gegossen und dreimal mit Aethylacetat extrahiert. Die vereinigten Essigesterextrakte werden mit Wasser gewaschen, über Natriumsulfat getrocknet und im Vakuum bei 40°C eingedampft. Der rote Eindampfrückstand wird mittels Säulenchromatographie an Kieselgel mit Aethylacetat als Elutionsmittel gereinigt. Umkristallisieren aus Aethylacetat/Aether/Petroläther liefert gelbliches 3-[(Diphenylmethyl)thio]-6-methoxy-2-methyl-as-triazin-5(2H)-on vom Schmelzpunkt 135-138°C.

e) Herstellung von 3,4-Dihydro-6-methoxy-2-methyl-3-thioxo-as-triazin-5(2H)-on

2,6 g 3-[(Diphenylmethyl)thio]-6-methoxy-2-methyl-as-triazin-5(2H)-on werden in 26 ml Anisol und 50 ml Trifluoressigsäure gelöst und 3 Stunden bei 25°C gerührt. Die Reaktionslösung wird im Vakuum bei 40°C eingedampft. Der Eindampfrückstand wird mit wenig Aethylacetat und viel tiefsiedendem Petroläther verrührt, abgenutscht und aus Methanol umkristallisiert. Man erhält 3,4-Dihydro-6-methoxy-2-methyl-3-thioxo-as-triazin-5(2H)-on als farblose, glänzende Kristalle vom Schmelzpunkt 215-220°C.

f) Herstellung von (6R,7R)-7-Amino-3-/[(2,5-dihydro-6-methoxy-2-methyl-5-oxo-as-triazin-3-yl)thio]-methyl/-8-oxo-5-thia-1-azabicyclo[4.2.0]oct-2-en-2-carbonsäure

13,6 g 7-Aminocephalosporansäure und 9,53 g 3,4-Dihydro-6-methoxy-2-methyl-3-thioxo-as-triazin-5(2H)-on werden in 250 ml Wasser suspendiert. Der pH-Wert der Suspension wird bei 25°C mit 1N Natronlauge auf 6,5 gestellt. Die Suspension wird auf 60°C erwärmt und unter Stickstoffbegasung 4 Stunden bei 60°C gerührt, wobei der pH-Wert mit 1N Natronlauge bei 6,5 gehalten wird. Nach 1 Stunde entsteht eine Lösung, nach 2 Stunden beginnt etwas Material auszufallen, und es wird keine Natronlauge mehr verbraucht. Das Gemisch wird auf 25°C abgekühlt und von wenig Ungelöstem mittels Filtration abgetrennt. Das orange Filtrat wird mit konz. Salzsäure bei 25°C auf pH 3,5 gestellt. Die ausgefallene Substanz wird abgenutscht, nacheinander mit 100 ml Wasser, 300 ml Aceton und 300 ml tiefsiedendem Petroläther gewaschen und über Nacht im Vakuum bei 40°C getrocknet. Man erhält eine beige gefärbte Substanz, die zur Reinigung in 150 ml Wasser/Methanol 1:1 suspendiert und mit 3,6 ml Triäthylamin tropfenweise während 1 Stunde versetzt wird, wobei bei pH 8,5 eine dunkle Lösung entsteht, welche noch wenig Ungelöstes enthält. Nach Zugabe von 3 g Entfärbungskohle wird 15 Minuten bei 25°C gerührt, über einen Hartfilter von der Entfärbungskohle abfiltriert und mit 50 ml Wasser/Methanol 1:1 nachgewaschen. Das orange Filtrat wird mit ca. 2 ml konz. Salzsäure auf pH 3 gestellt. Die dabei ausgefallene Substanz wird abgenutscht, nacheinander mit 50 ml Wasser/Methanol 1:1, 100 ml Methanol, 50 ml Aether und 50 ml tiefsiedendem Petroläther gewaschen und über Nacht im Vakuum bei 40°C getrocknet. Man erhält reine, beige-farbene (6R,7R)-7-Amino-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure.

g) Herstellung des Natriumsalzes der (6R,7R)-7-/2-[2-(2-Chloracetamido)-4-thiazolyl]-2-[(Z)-methoxyimino]-acetamido/-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure

Zu 200 ml über Calciumchlorid getrocknetem Methylenchlorid werden 25 g Phosphorpentachlorid gegeben. Die Suspension wird unter Rühren auf -10°C abgekühlt, und während ca. 5 Minuten werden 46 ml N,N-Dimethylacetamid zugetropft, wobei die Temperatur auf -5°C ansteigt. Die Suspension wird auf -15°C gekühlt und 15 Minuten gerührt. Nach dem Abkühlen auf -20°C werden 33,3 g 2-(2-Chloracetamido-4-thiazolyl)-2-(Z)-methoxyimino-essigsäure zugegeben und 45 Minuten bei -15°C gerührt, wobei eine gelb-orange Lösung entsteht. Diese Lösung wird auf -30°Cgekühlt, mit 50 g Eis versetzt und 10 Minuten bei -5°C gerührt. Die das gebildete Säurechlorid enthaltende Methylenchlorid-Phase wird abgetrennt und zunächst bei ca. -15°C aufbewahrt. Diese Säurechlorid-Lösung wird einer auf 0-5°C gekühlten Lösung, die durch Versetzen der unter f) hergestellten (6R,7R)-7-Amino-3-/[(2,5-dihydro-6-methoxy-2-methyl-5-oxo-as-triazin-3-yl)thio]methyl/-8-oxo-5-thia-1-azabicyclo-[4.2.0]oct-2-en-2-carbonsäure mit 3N Natronlauge bis pH 8,2 in 500 ml Wasser hergestellt wurde, während 30 Minuten unter kräftigem Rühren zugetropft, wobei der pH-Wert des mit 3N Natronlauge mit Hilfe eines Autotitrators bei 8,0-8,2 gehalten wird. Das Gemisch wird 2 Stunden bei 25°C gerührt. Danach werden 300 ml Methylenchlorid und 2 l n-Butanol zugegeben. Unter kräftigem Rühren wird das pH mit 3N Salzsäure auf 2 zurückgestellt. Die organische Phase wird abgetrennt, dreimal mit je 1 l Wasser gewaschen, mit 20 g Entfärbungskohle 15 Minuten lang verrührt und filtriert. Das hellgelbe Filtrat wird im Vakuum bei 60°C stark eingeengt, wobei das Reaktionsprodukt ausfällt. Letzteres wird abgenutscht und nacheinander mit n-Butanol, Aether und tiefsiedendem Petroläther gewaschen und über Nacht im Vakuum bei 40°C getrocknet. Man erhält rohe, beige (6R,7R)-7-/2-[2-(2-Chloracetamido)-4-thiazolyl]-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure. Zwecks Ueberführung in das Natriumsalz und Reinigung wird letztere in einem Gemisch von 300 ml Aceton und 50 ml

Methanol gelöst und mit 35 ml einer 2N Lösung von 2-Aethylcapronsäure-Natriumsalz in Essigester versetzt. Das ausgefallene Natriumsalz wird abgenutscht, nacheinander mit Aceton und tiefsiedendem Petroläther gewaschen und im Vakuum bei 25°C getrocknet. Man erhält beige-farbenes Natriumsalz der (6R,7R)-7-/2-[2-(2-Chloracetamido)-4-thiazolyl]-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo[4.2.0]oct-2-en-2-carbonsäure mit $[\alpha]_D^{25} = -131,5^\circ$ (c = 1 in Wasser).

## Beispiel 2

Herstellung des Dinatriumsalzes der (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]oct-2-en-2-carbonsäure

53 g (6R,7R)-3-/[(3-Carboxy-1-methyl-1H-1,2,4-triazol-5-yl)thio]methyl/-7-/2-[2-(2-chloracetamido)-4-thiazolyl]-2-[(Z)-methoxyimino]acetamido/-8-oxo-5-thia-1-azabicyclo[4.2.0]oct-2-en-2-carbonsäure werden in 1 l Wasser zusammen mit 34 g Thioharnstoff suspendiert. Das pH wird durch Zutropfen von 1N Natronlauge auf 7 gestellt, wobei eine dunkelbraune Lösung entsteht. Diese wird über Nacht unter Stickstoffbegasung bei pH 7 gerührt, wobei das pH mit 1N Natronlauge mit Hilfe eines Autotitrators konstant gehalten wird. Das pH der Reaktionslösung wird mit 1N Salzsäure auf 3,5 unter Rühren zurückgestellt. Die ausgefallene Substanz (Fraktion I) wird abgenutscht, mit 250 ml Wasser gewaschen und im Vakuum bei 40°C getrocknet. Die Fraktion I ist braun gefärbt und stark verunreinigt; sie wird verworfen. Die orange Mutterlauge wird mit 1N Salzsäure unter Rühren auf pH 2,65 zurückgestellt. Das ausgefallene Material wird abgenutscht und mit 250 ml Wasser gewaschen. Das beige-farbene Nutschgut wird unter vermindertem Druck mit Aethanol azeotropiert,

abgenutscht, mit Aethanol und tiefsiedendem Petroläther gewaschen und im Vakuum bei 40°C getrocknet. Man erhält somit eine beige-braune Fraktion II, welche zwecks Reinigung und Ueberführung in das Dinatriumsalz in 2 l Methanol suspendiert, mit 50 ml einer 2N Lösung des Natriumsalzes der 2-Aethylcapronsäure in Aethylacetat versetzt und anschliessend mit 200 ml Wasser 30 Minuten gerührt wird. Wenig ungelöstes, braunes Material wird abfiltriert und verworfen. Vom orange gefärbten Filtrat wird im Vakuum bei 40°C ca. 1 l abgedampft, dann 1 l Aethanol zugegeben und im Vakuum auf ein Volumen von ca. 500 ml eingeengt. Vom dabei ausgefallenen, schmierigen braunen Harz, welches ein starkes Gemisch darstellt, wird abdekantiert. Die abdekantierte gelborange Lösung wird mit 1 l Aethanol versetzt und im Vakuum bei 40°C stark eingeengt, wobei teilweise Substanz ausfällt. Nach Zugabe von 2,5 l Methanol entsteht eine gelbe Lösung, welche im Vakuum bei 40°C stark eingeengt wird. Das dabei ausgefallene Dinatriumsalz wird abgenutscht, mit Methanol und tiefsiedendem Petroläther gewaschen und im Hochvakuum bei 35°C getrocknet. Man erhält reines, beige-farbenes Dinatriumsalz der (6R,7R)-7-/2-(2-Amino-4-thiazolyl)-2-[(Z)-methoxyimino]acetamido/-3-/[(3 -carboxy-1-methyl-1H-1,2,4-triazol-5-yl)thio]methyl/-8-oxo-5-thia-1-azabicyclo[4.2.0]oct-2-en-2-carbonsäure mit $[\alpha]_D^{25} = -38,7°$ (c = 1 in Wasser).

Die im obigen Verfahren als Ausgangsmaterial verwendete Verbindung kann wie folgt hergestellt werden:

a) Herstellung von 4,5-Dihydro-1-methyl-5-thioxo-1H-1,2,4-triazol-3-carbonsäure-methylester

12,9 g 1-Methoxyoxalyl-2-methyl-thiosemicarbazid werden einer Lösung von 1,6 g Natrium in 200 ml Methanol zugegeben. Das Gemisch wird 4 Stunden unter Rückfluss gekocht. Die nach einer 1/2 Stunde ausgefallene Substanz

wird abgetrennt. Die Mutterlauge wird im Vakuum bei 40°C eingedampft. Der Eindampfrückstand wird in 100 ml Wasser gelöst und mit konz. Salzsäure angesäuert. Das dabei ausgeschiedene Kristallisat liefert nach dem Umkristallisieren aus Wasser reinen, farblosen 4,5-Dihydro-1-methyl-5-thioxo-1H-1,2,4-thiazol-3-carbonsäure-methylester vom Schmelzpunkt 188-190°C (Zers.).

b) Herstellung von 4,5-Dihydro-1-methyl-5-thioxo-1H-1,2,4-triazol-3-carbonsäure

3,46 g 4,5-Dihydro-1-methyl-5-thioxo-1H-1,2,4-triazol-3-carbonsäure-methylester werden in 50 ml 1N Natronlauge gelöst.Nach halbstündigem Rühren bei 25°C wird die Reaktionslösung mit 25 ml 2N Salzsäure sauer gestellt und im Vakuum bei 40°C eingeengt, wobei die gewünschte Säure kristallisiert. Diese wird abgenutscht, mit Eiswasser gewaschen und im Hochvakuum bei 45°C getrocknet. Man erhält farblose 4,5-Dihydro-1-methyl-5-thioxo-1H-1,2,4-triazol-3-carbonsäure vom Schmelzpunkt 177-178°C (Zers.).

c) Herstellung von (6R,7R)-7-Amino-3-/[(3-Carboxy-1-methyl-1,2,4-triazol-5-yl)thio]methyl/-8-oxo-5-thia-1-azabicyclo[4.2.0]oct-2-en-2-carbonsäure

34 g 7-Aminocephalosporansäure werden zusammen mit 25,5 g 4,5-Dihydro-1-methyl-5-thioxo-1H-1,2,4-triazol-3-carbonsäure in 500 ml Wasser suspendiert. Das pH wird mit 3N Natronlauge auf 6,5 gestellt. Das Gemisch wird 4 Stunden bei 55°C unter Stickstoffbegasung gerührt, wobei das pH mit 3N Natronlauge mit Hilfe eines Autotitrators konstant bei 6,5 gehalten wird. Die dunkle Lösung wird auf 25°C gekühlt und mit konz. Salzsäure auf pH 3,5 gestellt. Nach 15 Minuten wird das ausgefallene Material abgenutscht, in 500 ml Wasser suspendiert und mit 3N Natronlauge bei pH 7 in Lösung gebracht. Die orangerote

0036652

Lösung wird mit konz. Salzsäure auf pH 3 gestellt. Die ausgefallene Substanz wird abgenutscht und nacheinander mit 250 ml Wasser, 500 ml Aceton, 500 ml tiefsiedendem Petroläther gewaschen und über Nacht im Vakuum bei 40-45°C getrocknet. Man erhält (6R,7R)-7-Amino-3-/[(3-Carboxy-1-methyl-1,2,4-triazol-5-yl)thio]methyl/-8-oxo-5-thia-1-azabicyclo[4.2.0]oct-2-en-2-carbonsäure als beige gefärbtes Pulver.

d) Herstellung von (6R,7R)-3-/[(3-Carboxy-1-methyl-1H-1,2,4-triazol-5-yl)thio]methyl/-7-/2-[2-(2-chloracetamido)-4-thiazolyl]-2-[(Z)-methoxyimino]acetamido/-8-oxo-5-thia-1-azabicyclo[4.2.0]oct-2-en-2-carbonsäure

Diese Verbindung wird gemäss Absatz g) von Beispiel 1 ausgehend von 100 g 2-(2-Chloracetamido-4-thiazolyl)-2-(Z)-methoxyimino-essigsäure und 111,5 g (6R,7R)-7-Amino-3-/[)3-carboxy-1-methyl-1,2,4-triazol-5-yl)thio]methyl/8-oxo-5-thia-1-azabicyclo[4.2.0]oct-2-en-2-carbonsäure hergestellt. Man erhält beige-farbene (6R,7R)-3-/[(3-Carboxy-1-methyl-1H-1,2,4-triazol-5-yl)thio]methyl/-7-/2-[2-(2-chloracetamido)-4-thiazolyl]-2-[(Z)-methoxyimino]acetamido/-8-oxo-5-thia-1-azabicyclo[4.2.0]oct-2-en-2-carbonsäure.

## Beispiel 3

Herstellung des Natriumsalzes der (6R,7R)-7-[2-(2-Amino-4-thiazolyl)-2-((Z)-methoxyimino)acetamido]-3-[[[2,5-dihydro-2-methyl-5-oxo-6-[(pivaloyloxy)methoxy]-as-triazin-3-yl]thio]methyl]-5-oxo-4-thia-1-azabicyclo[4.2.0]oct-2-en-2-carbonsäure.

13,5 g (6R,7R)-7-[4-Brom-1-[(Z)-methoxyimino]acetoacetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure und 2,96 g Thioharnstoff werden in 80 ml Aethanol gelöst. Die Lösung wird 2 Stunden bei 25°C gerührt und dann mit 22 ml einer 2N

Lösung von 2-Aethylcapronsäure-Natriumsalz in Aethylacetat versetzt, wobei sofort das Natriumsalz ausfällt. Nun wird mit Aethanol auf 1000 ml verdünnt. Es werden noch 500 ml Methanol hinzugefügt, wobei eine gelbbräunliche Lösung entsteht. Im Vakuum bei 40°C werden ca. 600 ml Lösungsmittel abdestilliert. Das dabei ausgefallene Material wird abgenutscht, mit Aethanol und tiefsiedendem Petroläther gewaschen und im Hochvakuum bei 40°C getrocknet. Man erhält eine bräunliche Fraktion I, welche verworfen wird.

Aus der Mutterlauge werden im Vakuum bei 40°C weitere 250 ml Lösungsmittel abdestilliert. Das dabei ausgefallene Material wird abgenutscht, mit Aethanol und tiefsiedendem Petroläther gewaschen und über Nacht im Hochvakuum bei 40°C getrocknet. Man erhält eine beige Fraktion II. Zwecks Reinigung wird diese Substanz in 100 ml Wasser gelöst und mit 2 l Aethanol versetzt. Diese Lösung wird im Vakuum bei 40°C stark eingeengt. Das ausgefallene Material wird abgenutscht, mit Aethanol und tiefsiedendem Petroläther gewaschen und im Hochvakuum bei 40°C getrocknet. Man erhält eine Fraktion III, die verworfen wird. Die Mutterlauge wird noch, zwecks Entfernung von Wasser, 2 mal mit je 1 l Aethanol versetzt und am Vakuum bei 40°C stark eingeengt. Zuletzt wird noch Aceton zugegeben. Die dabei ausgefallene Substanz wird abgenutscht, mit Aceton und tiefsiedendem Petroläther gewaschen und über Nacht im Hochvakuum bei 40°C getrocknet. Man erhält somit reine, nicht kristalline Titelsubstanz mit $[\alpha]_D^{25}$ = 124,6° (c = 1 in Wasser). Das Kernresonanzspektrum und die Mikroanalyse entsprechen der angegebenen Struktur.

Die in obigem Verfahren als Ausgangsmaterial verwendete (6R,7R)-7-[4-Brom-2-[(Z)-methoxyimino]acetoacetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure kann wie folgt hergestellt werden:

a) Herstellung von Tetrahydro-2-methyl-5,6-dioxo-3-thioxo-as-triazin-1(2H)-carbonsäure-benzylester:

39,79 g 1,2,5,6-Tetrahydro-5,6-dioxo-3-mercapto-2-methyl-as-triazin werden in 500 ml Dimethylformamid und mit 35 ml Triäthylamin versetzt. Während 15 Minuten werden 39 ml Benzyloxycarbonylchlorid zugetropft, wobei die Temperatur des Reaktionsgemisches auf 45°C steigt, Triäthylamin-hydrochlorid ausfällt und die Suspension gelb gefärbt wird. Das Gemisch wird 2 1/2 Stunden bei 25°C gerührt und anschliessend am Vakuum bei 70°C eingedampft. Der ölige Eindampfrückstand wird mit 500 ml Wasser während 1 Stunde verrührt, wobei dieser fest wird. Der erhaltene Festkörper wird abgenutscht und mit 50 ml Wasser gewaschen. Das gelbe Nutschgut wird mit 250 ml Aethanol gut verrührt, abgenutscht, mit Aethanol gewaschen und im Vakuum bei 40°C getrocknet. Man erhält farbloses Rohkristallisat, das aus Methanol umkristallisiert wird und farblose Reinsubstanz vom Smp. 150-153°C liefert.

b) Herstellung von 3-[(Diphenylmethyl)thio]-5,6-dihydro-2-methyl-5,6-dioxo-as-triazin-1(2H)-carbonsäure-benzylester:

13,2 g Tetrahydro-2-methyl-5,6-dioxo-3-thioxo-as-triazin-1(2H)-carbonsäure-benzylester werden in 500 ml Essigester gelöst und mit einer Lösung von Diphenyldiazomethan in 90 ml tiefsiedendem Petroläther versetzt. Die anfangs violette Reaktionslösung wird 40 Stunden bei 25°C stehen gelassen, wobei die Farbe blassrot wird. Es werden 3 ml Eisessig zugegeben, und das Gemisch wird nach 1 Stunde im Vakuum bei 40°C eingedampft. Man erhält ein gelbes Oel als Eindampfrückstand. Dieser wird mittels Säulenchromatographie an Kieselgel mit den Elutionsmitteln Benzol, Benzol/Aethylacetat 95:5 und Benzol/Aethylacetat 90:10 aufgetrennt. Die die gewünschte Substanz enthaltenden Fraktionen werden gesammelt und im Vakuum bei 40°C eingedampft. Man erhält, nach dem Umkristallisieren aus

Aethanol, farblose Reinsubstanz vom Smp. 90-92°C.

c) Herstellung von 3-[(Diphenylmethyl)thio]-1,2-dihydro-2-methyl-as-triazin-5,6-dion:

6,9 g der unter b) hergestellten Substanz werden in 100 ml Aethylacetat gelöst und mit 30 ml einer wässrigen, 7%igen Ammoniak-Lösung bei 25°C während 15 Minuten gut durchgerührt. Die Aethylacetatphase wird abgetrennt und verworfen. Die wässrige Phase wird mit 7 ml konz. Salzsäure versetzt und 30 Minuten im Eisbad gerührt. Die dabei ausgefallene Substanz wird abgenutscht, mit Wasser gewaschen und sofort aus Aethanol umkristallisiert. Man erhält farblose Reinsubstanz vom Smp. 180-182°C.

d) Herstellung von 3-[(Diphenylmethyl)thio]-6-pivaloyloxymethoxy-2-methyl-as-triazin-5(2H)-on:

3,25 g der unter c) hergestellten Verbindung werden in 40 ml Dimethylformamid gelöst und mit 1,52 g Kaliumcarbonat versetzt. Zu diesem Gemisch werden auf einmal 2,66 g Pivaloyloxymethyljodid gegeben, wobei eine blassgelbe Lösung entsteht. Das Reaktionsgemisch wird 4 Stunden bei 25°C gerührt und noch einmal mit 2,66 g Pivaloyloxymethyljodid versetzt. Dieses Gemisch wird 15 Stunden bei 25°C gerührt und anschliessend im Hochvakuum bei 35°C eingedampft. Der resultierende Eindampfrückstand wird zwischen 100 ml Wasser und 100 ml Aethylacetat verteilt. Die wässrige Phase wird noch zweimal mit je 50 ml Aethylacetat extrahiert. Die vereinigten Aethylacetat-Phasen werden über Natriumsulfat getrocknet und im Vakuum bei 35°C eingedampft. Das verbleibende gelbe Oel wird an einer Kieselgelsäule mit Aethylacetat als Elutionsmittel chromatographiert. Die das gewünschte Produkt enthaltenden Fraktionen werden gesammelt und im Vakuum bei 35°C eingedampft. Das verbleibende blassblaue Oel wird im Hochvakuum bei 25°C während 1 Stunde getrocknet, wobei man die Reinsubstanz als blassblaues Harz erhält.

e) Herstellung von [(2,3,4,5-Tetrahydro-2-methyl-5-oxo-3-thioxo-as-triazin-6-yl)oxy]methyl pivalat:

3,6 g der unter d) hergestellten Verbindung werden in 18 ml Anisol und 18 ml Trifluoressigsäure 2 1/2 Stunden bei 25°C gerührt. Die Lösung wird dann im Vakuum bei 50°C eingedampft. Der ölige Rückstand wird mit 50 ml tiefsiedendem Petroläther verrührt, wobei Kristallisation eintritt. Das Kristallisat wird abgenutscht und mit tiefsiedendem Petroläther gewaschen. Man erhält weisse Kristalle, die nach dem Umkristallisieren aus Aether/tiefsiedendem Petroläther weisse, kristalline Reinsubstanz vom Smp. 112-113°C liefern. Das Kernresonanzspektrum und die Mikroanalyse entsprechen der angegebenen Struktur.

f) Herstellung von (6R,7R)-7-Amino-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure:

3,0 g der unter e) hergestellten Verbindung werden zusammen mit 2,72 g 7-Aminocephalosporansäure in 50 ml Wasser suspendiert. Die Suspension wird unter Stickstoff-Begasung mit 1N Natronlauge auf pH 6,5 gestellt und 4 Stunden bei pH 6,5-7 und 55-60°C gerührt. Die gewünschte Verbindung fällt aus und wird nach dem Abkühlen des Gemisches auf 25°C abgenutscht. Nach dem Waschen mit Wasser, Aceton und tiefsiedendem Petroläther und Trocknen über Nacht im Hochvakuum bei 40°C erhält man die Reinsubstanz, deren Mikroanalyse und Kernresonanzspektrum der angegebenen Struktur entsprechen.

g) Silylierung von (6R,7R)-7-Amino-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure:

14,56 g der unter f) hergestellten Verbindung werden in 300 ml Aethylacetat suspendiert und mit 30 ml N,O-Bis-(trimethylsilyl)-acetamid versetzt. Das Gemisch wird 30 Minuten bei 35-40°C gerührt, wobei eine dunkle Lösung entsteht. Diese Lösung wird auf -5° bis -10°C gekühlt und bis zur Acylierung unter Feuchtigkeitsausschluss gerührt.

h) Herstellung von (Z)-2-Hydroxyimino-3-oxo-buttersäure-tert.-butylester:

592,1 g Acetessigsäure-tert.-butylester werden in 560 ml Eisessig gelöst. Zu dieser Lösung wird bei 5-10°C während 2 1/2 Stunde eine Lösung von 290,6 g Natriumnitrit in 655 ml Wasser getropft. Die entstandene gelbe Suspension wird 30 Minuten bei 20°C gerührt, mit 940 ml Wasser versetzt und weitere 2 Stunden gerührt. Das Gemisch wird mit 900 ml Wasser und 900 g Eis versetzt und im Ausrührgefäss dreimal mit je 1 l Aethylacetat extrahiert. Die vereinigten Aethylacetatextrakte werden dreimal mit je 1 l Wasser gewaschen, dann mit 5 l Wasser versetzt und mit Natriumhydrogencarbonat der pH-Wert auf 6,8 gestellt. Nach dem Abtrennen der wässrigen Phase wird noch einmal mit Wasser gewaschen. Danach wird die Aethylacetatlösung über Natriumsulfat getrocknet und am Vakuum bei 40°C eingedampft. Man erhält (Z)-2-Hydroxyimino-3-oxo-buttersäure-tert.-butylester als gelbes Oel, das noch 9 Stunden am Hochvakuum bei 40°C getrocknet wird.

i) Herstellung von (Z)-2-Methoxyimino-3-oxo-buttersäure-tert.-butylester:

626,65 g (Z)-2-Hydroxyimino-3-oxo-buttersäure-tert.-butylester werden in 2,86 l Aceton gelöst. Die Lösung wird auf 5°C abgekühlt und portionenweise mit 703,5 g Kaliumcarbonat versetzt. Zur gelben Suspension werden dann ohne Kühlung während 1 Stunde 322 ml Dimethylsulfat hinzugetropft, wobei die Temperatur des Gemisches nicht über 25°C steigen soll. Die hellbeige Suspension

wird bei 20-25°C ca. 4 Stunden gerührt, bis dünnschicht-chromatoghraphisch kein Ausgangsmaterial mehr nachgewiesen wird. Danach wird das Gemisch auf 7 l Wasser gegossen und dreimal mit je 1 l Aethylacetat extrahiert. Die vereinigten Aethylacetatextrakte werden dreimal mit je 1 l Wasser gewaschen, über Natriumsulfat getrocknet und am Vakuum bei 40°C eingedampft. Das verbleibende, gelbe Oel wird noch 6 Stunden am Hochvakuum bei 40°C getrocknet und anschliessend destilliert. Man erhält (Z)-2-Methoxyimino-3-oxo-buttersäure-tert.-butylester als gelbes Oel mit einem Siedepunkt von 57°C bei 0,02 mm Hg.

j) Herstellung von (Z)-2-Methoxyimino-3-oxo-buttersäure:

86 g (Z)-2-Methoxyimino-3-oxo-buttersäure-tert.-butylester werden in 400 ml Trifluoressigsäure gelöst. Die Lösung wird 1 Stunde bei 25°C stehen gelassen, danach am Vakuum bei 35°C eingedampft. Der ölige Rückstand wird aus Aether/Petroläther kristallisiert. Man erhält gelbliche, wasserlösliche (Z)-2-Methoxyimino-3-oxo-buttersäure vom Smp. 80-85°C.

k) Herstellung von (Z)-4-Brom-2-methoxyimino-3-oxo-buttersäure:

145 g (Z)-2-Methoxyimino-3-oxo-buttersäure werden in 1000 ml alkohol- und wasserfreiem Dichlormethan gelöst. Zu dieser Lösung werden 10 ml 30 o/oige Bromwasserstoffsäure in Eisessig gegeben. Dann wird während ca. 2 Stunden eine Lösung von 37,5 ml Brom in 112,5 ml Dichlormethan zugetropft, wobei die Temperatur des Reaktionsgemisches mittels leichter Kühlung auf 20-25°C gehalten wird. Nun wird zur Entfernung von HBr aus dem Reaktionsgemisch heftig Stickstoff durchgeblasen. Anschliessend werden nacheinander 250 g Eis, 250 ml Wasser und 2 l. Aether zugegeben. Die wässrige Phase wird abgetrennt und verworfen. Die organische Phase wird mit 250 ml Wasser und 250 ml gesättigter Natriumchloridlösung gewaschen, mit Natriumsulfat getrocknet und im Vakuum eingedampft. Es verbleiben 170 g braunes Oel, das aus Tetrachlorkohlenstoff kristallisiert wird. Man erhält praktisch farblose (Z)-4-Brom-2-methoxyimino-3-oxo-buttersäure.

l) Herstellung des Säurechlorids der (Z)-4-Brom-2-methoxyimino-3-oxo-buttersäure:

6,72 g (Z)-4-Brom-2-methoxyimino-3-oxo-buttersäure werden in 70 ml Wasser- und Alkohol-freiem Methylenchlorid gelöst und bei 0-5°C mit 6,24 g Phosphorpentachlorid versetzt. Die Reaktionslösung wird 15 Minuten bei 0-5°C und 45 Minuten ohne Kühlung gerührt.

m) Herstellung von (6R,7R)-7-[4-Brom-2-[(Z)-methoxyimino]-acetoacetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carbonsäure:

Die nach l) hergestellte Lösung wird während ca. 30 Minuten zur nach g) hergestellten Lösung bei -10 bis 0°C zugetropft. Das Gemisch wird 30 Minuten bei -10 bis 0°C und 1 Stunde ohne Kühlung gerührt; dann werden 300 ml Aethylacetat und 200 ml Wasser zugegeben. Das ausgefallene Material wird abgenutscht und verworfen. Die wässrige Phase des Filtrats wird abgetrennt und verworfen; die organische Phase wird 6 mal mit je 250 ml Wasser gewaschen, mit Natriumsulfat getrocknet, im Vakuum bei 40°C stark eingeengt und schliesslich auf 500 ml Aether gegossen. Die ausgefallene Substanz wird abgenutscht, mit Aether und tiefsiedendem Petroläther gewaschen und im Hochvakuum bei 40°C über Nacht getrocknet. Man erhält beige, amorphe Reinsubstanz. Das Kernresonanzspektrum und das Infrarotspektrum entsprechen der angegebenen Struktur.

## Beispiel 4

Herstellung von Trockenampullen für die intramuskuläre Verabreichung:

Es wird in üblicher Weise ein Lyophilisat von 1 g des Dinatriumsalzes der (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]oct-2-en-2-carbonsäure hergestellt und in eine Ampulle abgefüllt. Vor der Verabreichung wird das Lyophilisat mit 2,5 ml einer 2%igen wässrigen Lidocainhydrochlorid-Lösung versetzt.

Patentansprüche

1. Cephalosporinderivate der allgemeinen Formel

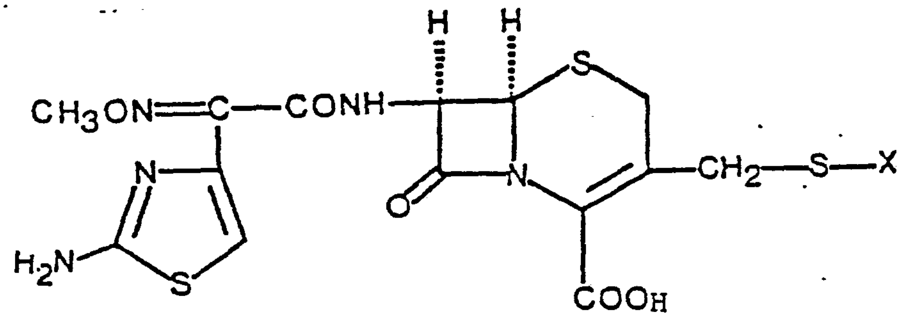

I

in der X eine der Gruppen

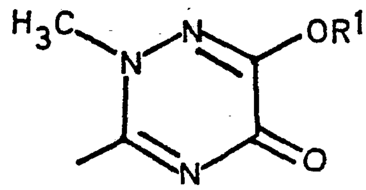

(a)

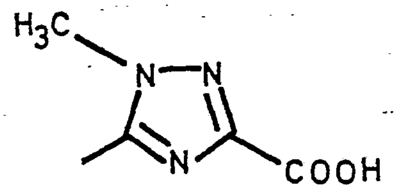

(b)

worin $R^1$ niederes Alkyl oder (zusammen mit dem Sauerstoff) eine leicht hydrolysierbare Aethergruppe darstellt, ist sowie leicht hydrolysierbare Ester der Verbindungen der Formel I, worin X die Gruppe (b) oder (a) mit $R^1$=niederes Alkyl bedeutet und Salze dieser Verbindungen und Hydrate der Verbindungen der Formel I bzw. von deren Estern und Salzen.

2. Cephalosporinderivate gemäss Anspruch 1, dadurch gekennzeichnet, dass X die Gruppe (a) mit $R^1$=Methyl oder die Gruppe (b) darstellt.

3. Cephalosporinderivate gemäss Anspruch 1, dadurch gekennzeichnet, dass X die Gruppe (a) mit $R^1$=Pivaloyloxymethyl darstellt.

4. (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo-[4.2.0]oct-2-en-2-carbonsäure sowie Salze dieser Verbindung und Hydrate dieser Verbindung bzw. Salze.

5. (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]-oct-2-en-2-carbonsäure sowie Salze dieser Verbindung und Hydrate dieser Verbindung bzw. Salze.

6. (6R,7R)-7-[2-(2-Amino-4-thiazolyl)-2-((Z)-methoxyimino)acetamido]-3-[[[2,5-dihydro-2-methyl-5-oxo-6-[(pivaloyloxy)methoxy]-as-triazin-3-yl]thio]methyl]-5-oxo-4-thia-1-azabicyclo[4.2.0]oct-2-en-2-carbonsäure sowie Salze dieser Verbindung und Hydrate dieser Verbindung bzw. Salze.

7. Cephalosporinderivate der allgemeinen Formel

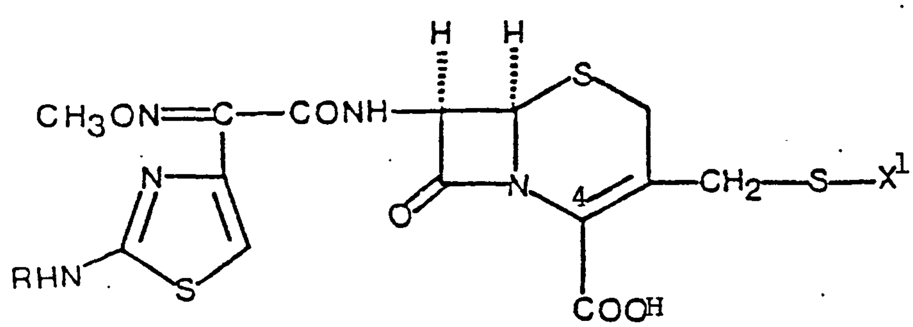

II

in der $X^1$ eine der Gruppen

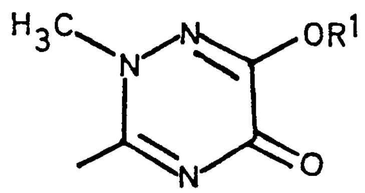

(a)

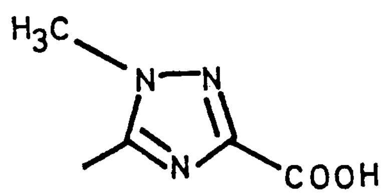

(b)

ist,

worin $R^1$ niederes Alkyl oder (zusammen mit dem Sauerstoff) eine leicht hydrolysierbare Aethergruppe darstellt, wobei die Carboxygruppe des Restes (b) geschützt sein kann, R eine abspaltbare Schutzgruppe darstellt und die Carboxygruppe in 4-Stellung des Cephalosporingerüsts in geschützter Form vorliegen kann.

8. Verbindungen der allgemeinen Formel

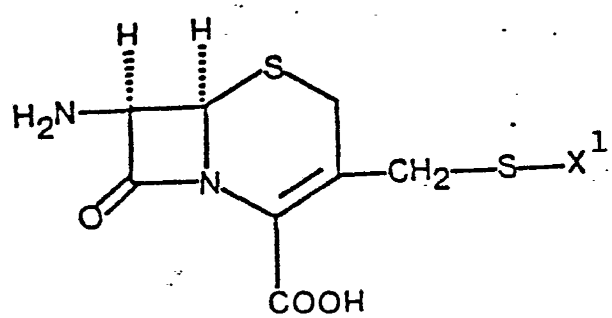

IV

in der $X^1$ eine der Gruppen

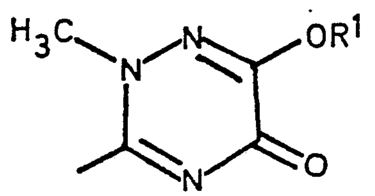

(a)

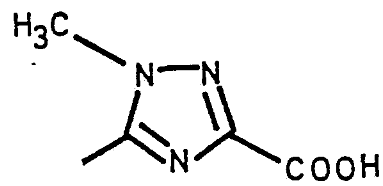

(b)

ist,

worin $R^1$ niederes Alkyl oder (zusammen mit dem Sauerstoff) eine leicht hydrolysierbare Aethergruppe darstellt, wobei die Carboxygruppe des Restes (b) geschützt sein kann und die Carboxygruppe in 4-Stellung des Cephalosporingerüsts und/oder die Aminogruppe in geschützter Form vorliegen kann.

9. Halogenide der allgemeinen Formel

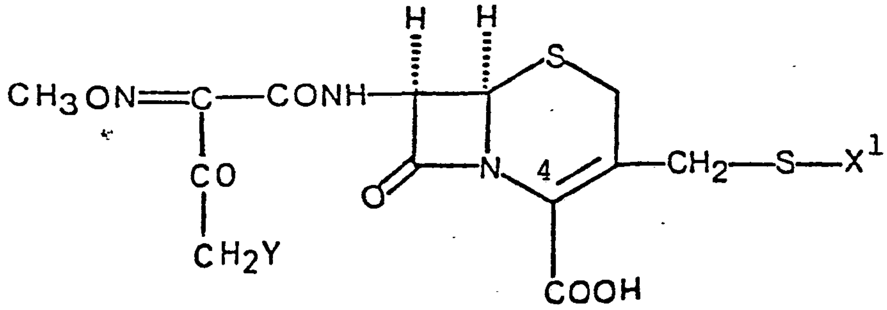

III

in der $X^1$ eine der Gruppen

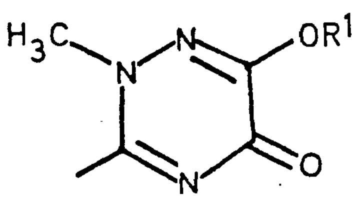

(a)

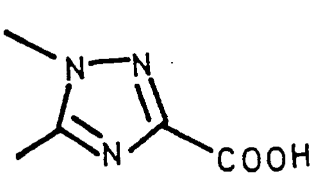

(b)

ist,
worin Y ein Halogenatom und $R^1$ niederes Alkyl oder (zusammen mit dem Sauerstoff) eine leicht hydrolysierbare Aethergruppe darstellt, wobei die Carboxygruppe des Restes (b) geschützt sein kann, und die Carboxygruppe in 4-Stellung des Cephalosporingerüsts in geschützter Form vorliegen kann.

10. Verbindungen der tautomeren Formeln

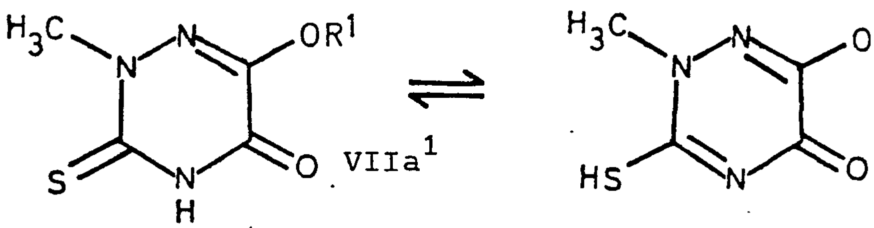

$VIIa^1$ $VIIa^2$

worin $R^1$ niederes Alkyl oder (zusammen mit dem Sauerstoff) eine leicht hydrolysierbare Aethergruppe darstellt.

11. Eine Verbindung der tautomeren Formeln

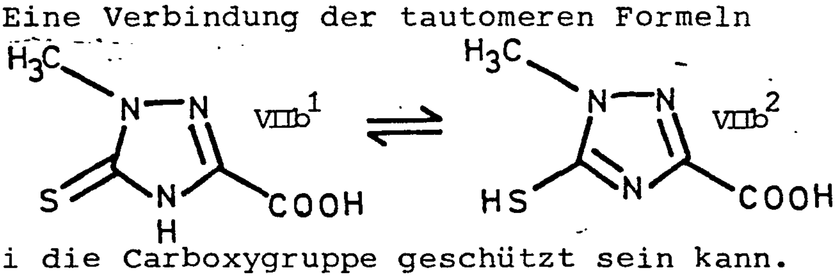

$VIIb^1$ $VIIb^2$

wobei die Carboxygruppe geschützt sein kann.

12. Verbindungen gemäss einem der Ansprüche 1-6 als pharmazeutische Wirkstoffe.

13. Verbindungen gemäss einem der Ansprüche 1-6 als pharmazeutische Wirkstoffe zur Behandlung und Prophylaxe von Infektionskrankheiten.

14. Pharmazeutische Präparate, gekennzeichnet durch einen Gehalt an einer Verbindung gemäss einem der Ansprüche 1-6.

15. Pharmazeutische Präparate zur Behandlung und Prophylaxe von Infektionskrankheiten, gekennzeichnet durch einen Gehalt an einer Verbindung gemäss einem der Ansprüche 1-6.

16. Verfahren zur Herstellung der Verbindungen gemäss einem der Ansprüche 1-6, dadurch gekennzeichnet, dass man

a) in einer Verbindung der allgemeinen Formel

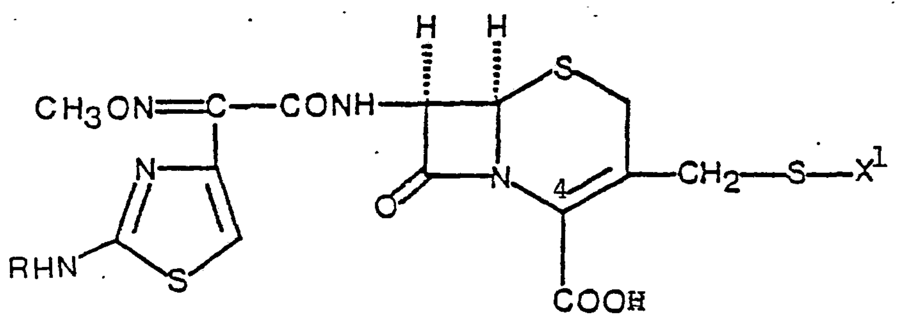

II

in der $X^1$ die gleiche Bedeutung wie X in Anspruch 1 hat, wobei die Carboxygruppe des Restes (b) geschützt sein kann, R eine abspaltbare Schutzgruppe darstellt und die Carboxygruppe in 4-Stellung des Cephalosporingerüsts in geschützter Form vorliegen kann,

die Schutzgruppe R, ggfs. auch allenfalls vorhandene Carboxyschutzgruppen, abspaltet, oder dass man

b) ein Halogenid der allgemeinen Formel

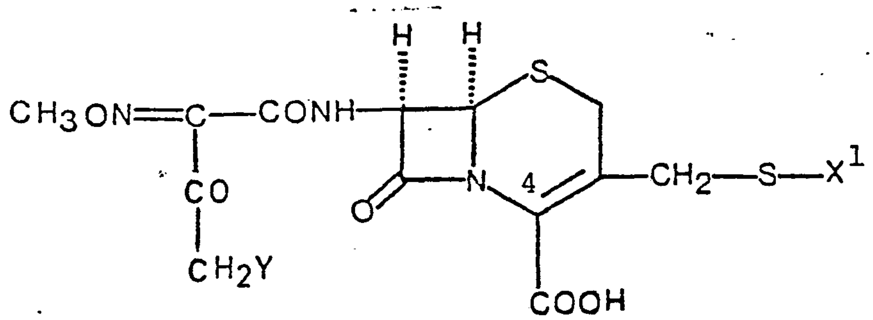

III

in der $X^1$ die oben gegebene Bedeutung hat und Y ein Halogenatom darstellt und die Carboxygruppe in 4-Stellung des Cephalosporingerüsts in geschützter Form vorliegen kann,

mit Thioharnstoff umsetzt, oder dass man

c) zur Herstellung eines leicht hydrolysierbaren Esters einer Verbindung der Formel I eine Carbonsäure der Formel I einer entsprechenden Veresterung unterwirft, oder dass man

d) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

17. Verfahren nach Anspruch 16, dadurch gekennzeichnet, dass man eine Ausgangsverbindung der Formel II oder III, worin X die Gruppe (a) mit $R^1$=Methyl oder die Gruppe (b) darstellt, einsetzt.

18. Verwendung von Verbindungen gemäss einem der Ansprüche 1-6 bei der Behandlung bzw. Prophylaxe von Krankheiten.

19. Verwendung von Verbindungen gemäss einem der Ansprüche 1-6 bei der Behandlung bzw. Prophylaxe von Infektionskrankheiten.

***

Patentansprüche
"für OESTERREICH"

1. Verfahren zur Herstellung von Cephalosporinderivaten der allgemeinen Formel

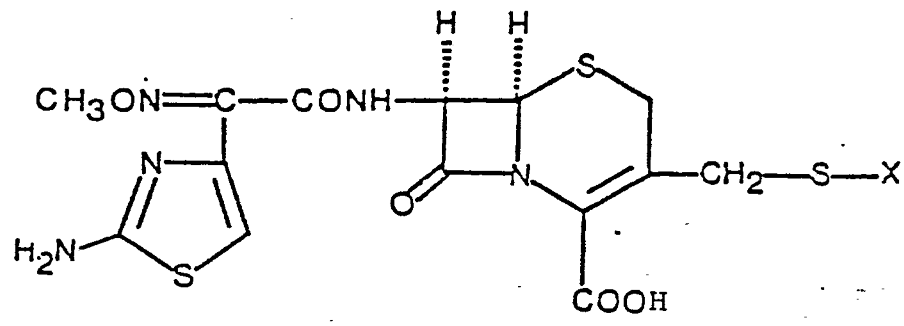

I

in der X eine der Gruppen

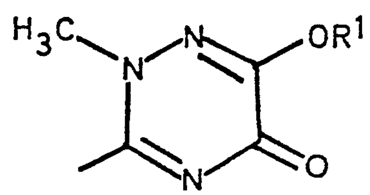

(a)

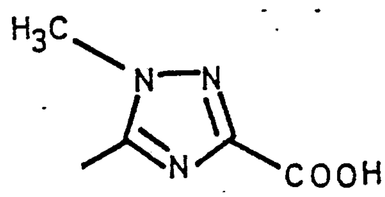

(b)

worin $R^1$ niederes Alkyl oder (zusammen mit dem Sauerstoff) eine leicht hydrolysierbare Aethergruppe darstellt, ist sowie leicht hydrolysierbare Ester der Verbindungen der Formel I, worin X die Gruppe (b) oder (a), worin $R^1$ niederes Alkyl darstellt, bedeutet und Salze dieser Verbindungen und Hydrate der Verbindungen der Formel I bzw. von deren Estern und Salzen, dadurch gekennzeichnet, dass man

a) in einer Verbindung der allgemeinen Formel

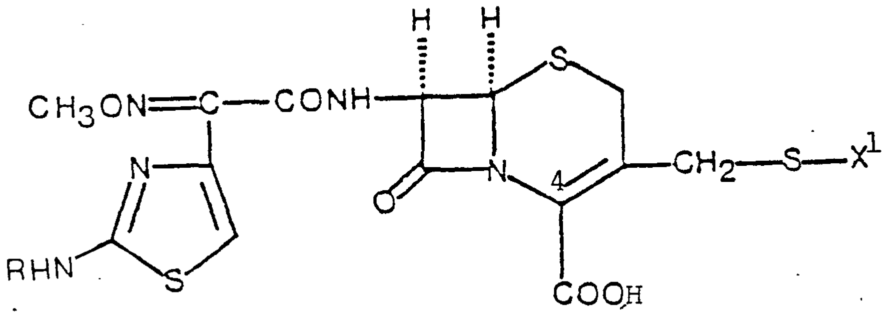

II

in der $X^1$ die gleiche Bedeutung wie X in Formel I hat, wobei die Carboxygruppe des Restes (b) geschützt sein kann, R eine abspaltbare Schutzgruppe darstellt und die Carboxygruppe in 4-Stellung des Cephalosporingerüsts in geschützter Form vorliegen kann,

die Schutzgruppe R, ggfs. auch allenfalls vorhandene Carboxyschutzgruppen, abspaltet, oder dass man

b) ein Halogenid der allgemeinen Formel

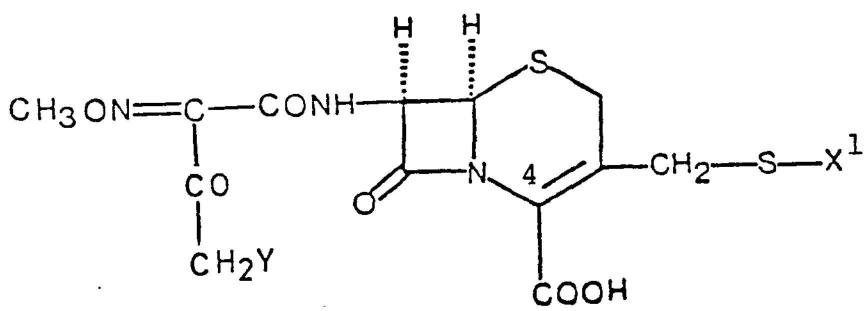

III

in der $X^1$ die oben gegebene Bedeutung hat und Y ein Halogenatom darstellt und die Carboxygruppe in 4-Stellung des Cephalosporingerüsts in geschützter Form vorliegen kann,

mit Thioharnstoff umsetzt, oder dass man

c) zur Herstellung eines leicht hydrolysierbaren Esters einer Verbindung der Formel I eine Carbonsäure der Formel I einer entsprechenden Veresterung unterwirft, oder dass man

d) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

2. Verfahren zur Herstellung von Cephalosporinderivaten gemäss Anspruch 1, worin X die Gruppe (a) mit $R^1$=Methyl oder die Gruppe (b) darstellt, dadurch gekennzeichnet, dass man entsprechend substituierte Ausgangsverbindungen der Formeln II oder I einsetzt.

3. Verfahren zur Herstellung von Cephalosporinderivaten gemäss Anspruch 1, worin X die Gruppe (a) mit $R^1$=Pivaloyloxymethyl darstellt, dadurch gekennzeichnet, dass man entsprechend substituierte Ausgangsverbindungen der Formeln II, III oder I einsetzt.

4. Verfahren nach Anspruch 1 zur Herstellung von (6R,7R)-7-/2-(2-Amino-4-thiazolyl)-2-[(Z)-methoxyimino]acetamido/-3-/[(2,5-dihydro-6-methoxy-2-methyl-5-oxo-as-triazin-3-yl)thio]methyl/-8-oxo-5-thia-1-azabicyclo-[4.2.0]oct-2-en-2-carbonsäure sowie von Salzen dieser Verbindung und von Hydraten dieser Verbindung bzw. Salze, dadurch gekennzeichnet, dass man entsprechend substituierte Ausgangsverbindungen der Formeln II oder I einsetzt.

5. Verfahren nach Anspruch 1 zur Herstellung von (6R,7R)-7-/2-(2-Amino-4-thiazolyl)-2-[(Z)-methoxyimino]acetamido/-3-/[(2,5-dihydro-6-methoxy-2-methyl-5-oxo-triazin-3-yl)thio]methyl/-8-oxo-5-thia-1-azabicyclo-[4.2.0]oct-2-en-2-carbonsäure sowie von Salzen dieser Verbindung und von Hydraten dieser Verbindung bzw. Salze, dadurch gekennzeichnet, dass man eine entsprechend substituierte Ausgangsverbindung der Formel III einsetzt.

6. Verfahren nach Anspruch 1 zur Herstellung von (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]-oct-2-en-2-carbonsäure sowie von Salzen dieser Verbindung und von Hydraten dieser Verbindung bzw. Salze, dadurch gekennzeichnet, dass man entsprechend substituierte Ausgangsverbindungen der Formeln II oder I einsetzt.

7. Verfahren nach Anspruch 1 zur Herstellung von (6R,7R)-7-/2-(2-Amino-4-thiazolyl)-2-[(Z)-methoxyimino]acetamido/-3-/[(3-carboxy-1-methyl-1H-1,2,4-triazol-5-yl)thio]methyl/-8-oxo-5-thia-1-azabicyclo[4.2.0]-oct-2-en-2-carbonsäure sowie von Salzen dieser Verbindung und von Hydraten dieser Verbindung bzw. Salze, dadurch gekennzeichnet, dass man eine entsprechend substituierte Ausgangsverbindung der Formel III einsetzt.

8. Verfahren nach Anspruch 1 zur Herstellung von (6R,7R)-7-[2-(2-Amino-4-thiazolyl)-2-((Z)-methoxyimino)acetamido]-3-[[[2,5-dihydro-2-methyl-5-oxo-6-[(pivaloyloxy)methoxy]-as-triazin-3-yl]thio]methyl]-5-oxo-4-thia-1-azabicyclo[4.2.0]oct-2-en-2-carbonsäure sowie von Salzen dieser Verbindung und von Hydraten dieser Verbindung bzw. Salze, dadurch gekennzeichnet, dass man entsprechend substituierte Ausgangsverbindungen der Formeln II, III oder I einsetzt.